# EUROPÄISCHE PATENTSCHRIFT

(11) **EP 1 663 976 B1**
(45) Veröffentlichungstag und Bekanntmachung des Hinweises auf die Patenterteilung: **30.12.2009**
(21) Anmeldenummer: 04765053.6
(22) Anmeldetag: 10.09.2004
(51) Int. Cl.: C07D 213/42

(54) **4-PYRIDINYLMETHYLSULFONAMIDDERIVATE ALS FUNGIZIDWIRKSAME PFLANZENSCHUTZMITTEL**
4-PYRIDINYLMETHYL SULFONAMIDE DERIVATIVES AS FUNGICIDAL PLANT PROTECTION AGENTS
DERIVES DE 4-PYRIDINYLMETHYL SULFONAMIDE COMME AGENTS PHYTOSANITAIRES FONGICIDES

(30) Priorität: 18.09.2003 EP 03021098
(43) Veröffentlichungstag der Anmeldung: 07.06.2006
(73) Patentinhaber: BASF SE, 67056 Ludwigshafen (DE)
(72) Erfinder: GRAMMENOS, Wassilios, 67071 Ludwigshafen (DE); BLETTNER, Carsten, 68165 Mannheim (DE); MÜLLER, Bernd, 67227 Frankenthal (DE); GEWEHR, Markus, 56288 Kastellaun (DE); TORMO I BLASCO, Jordi, 69514 Laudenbach (DE); GROTE, Thomas, 67157 Wachenheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); WAGNER, Oliver, 67433 Neustadt (DE); GÖTZ, Norbert, 67547 Worms (DE); STRATHMANN, Siegfried, 67117 Limburgerhof (DE); SCHÖFL, Ulrich, 68782 Brühl (DE); SCHERER, Maria, 76829 Godramstein (DE); STIERL, Reinhard, 67251 Freinsheim (DE)
(86) Internationale Anmeldenummer: PCT/EP2004/010124
(87) Internationale Veröffentlichungsnummer: WO 2005/033081

(56) Entgegenhaltungen:
- EP-A- 0 206 581
- EP-A- 0 209 854
- EP-A- 1 174 028

## Beschreibung

Die vorliegende Erfindung betrifft Sulfonamide der Formel I 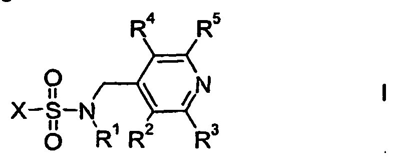 in der die Substituenten folgende Bedeutung haben:
- R¹: Wasserstoff, C₁-C₄-Alkyl, C₁-C₄-Alkoxy, C₂-C₄-Alkenyl, C₂-C₄-Alkinyl oder Benzyl;
- R², R³, R⁴, R⁵: unabhängig voneinander Wasserstoff, Halogen, C₁-C₄-Alkyl, C₁-C₄- Alkoxy oder C₁-Halogenmethyl;
R² und R³ können auch gemeinsam einen Phenyl-, Cyclopentyl- oder Cyclohexylring bilden, wobei diese Ringe zwei Gruppen R^{2,} und R^{3,} tragen können,
- R^{2,}, R^{3,}: unabhängig voneinander Wasserstoff, Halogen, C₁-C₄-Alkyl, C₁-C₄-Alkoxy oder C₁-Halogenmethyl;
im Fall a), wenn R², R³, R⁴ und R⁵ Wasserstoff bedeuten:
- X: Phenyl, welches durch eine Gruppe -C(R⁶)=NOR⁷, mit
R⁶ C₁-C₄-Alkyl und
R⁷ C₁-C₈-Alkyl, Benzyl, C₂-C₄-Alkenyl, C₁-C₄-Halogenalkyl, C₂-C₄-Halogen- alkenyl, C₂-C₄-Alkinyl oder C₂-C₄-Halogenalkinyl substituiert ist; und
im Fall b), wenn mindestens eine der Gruppen R², R³, R⁴ und R⁵ nicht Wasserstoff bedeutet
- X: Phenyl, Naphthyl oder ein fünf- oder sechsgliedriger über ein C-Atom gebunde- ner gesättigter, partiell ungesättigter oder aromatischer Heterocyclus, enthaltend ein bis vier Heteroatome aus der Gruppe O, N oder S, wobei X eine bis vier Gruppen R^{a} tragen kann:
R^{a} Halogen, Cyano, Nitro, C₁-C₈-Alkyl, C₁-C₈-Alkoxy, C₁-C₈-Halogenalkoxy, C₁-C₄-Alkylcarbonyl, C₁-C₄-Alkoxycarbonyl, -C(R⁶)=NOR⁷, C₁-C₄-Alkyl- aminocarbonyl, Di-(C₁-C₄-alkyl)aminocarbonyl oder Phenyl oder Phenoxy, wobei die Ringe eine bis drei Gruppen R^{b} tragen können:
R^{b} Halogen, C₁-C₄-Alkyl, C₁-C₄-Alkoxy, C₁-Halogenalkyl oder C₁-Halogenalkoxy,
R^{a} oder R^{b} kann auch eine C₃-C₄-Alkylen- oder eine C₄-Alkenylengruppe bedeuten, die mit zwei benachbarten Ringgliedern des Phenylringes, an den sie gebunden ist, einen Ring bilden, der durch eine oder meh- rere der voranstehend genannten Gruppen R^{a} oder R^{b} substituiert sein kann.

Außerdem betrifft die Erfindung Verfahren zur Herstellung dieser Verbindungen, sie enthaltende Mittel sowie ihre Verwendung zur Bekämpfung von pflanzenpathogenen Schadpilzen.

In DE-A 31 22 700 und WO 00/06083 werden Pyridinosulfonamide als Pharmaka allgemein offenbart. 4-Pyridinmethylsulfonamide sind von deren allgemeinen Offenbarung umfasst.

Aus EP-A 206 581 und Lieb. Ann. Chem. 641 (1990) sind einzelne 4-Pyridinmethylsulfonamide bekannt. Die in den genannten Schriften beschriebenen Verbindungen sind zur Bekämpfung von Schadpilzen geeignet.

Ihre Wirkung ist jedoch in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäss wurden die eingangs definierten Verbindungen gefunden. Des weiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die erfindungsgemäßen Verbindungen unterscheiden sich von den in EP-A 206 581, DE-A 31 22 700 und WO 00/06083 beschriebenen durch die 4-Pyridinomethylgruppe und von den aus Lieb. Ann. Chem. 641 (1990) bekannten Verbindungen durch die Substitution des Pyridinringes, bzw. der Gruppe X.

Die Verbindungen der Formel I weisen eine gegenüber den bekannten Verbindungen erhöhte Wirksamkeit gegen Schadpilze auf.

Die erfindungsgemäßen Verbindungen können auf verschiedenen Wegen erhalten werden. Vorteilhaft werden sie ausgehend von Pyridinderivaten der Formel II durch Umsetzung mit Sulfonsäuren oder aktivierten Sulfonsäurederivaten der Formel III, in der X die Bedeutung gemäß Anspruch 1 hat, unter basischen Bedingungen erhalten. In Formel III steht L für Hydroxy oder Halogen, bevorzugt Chlor.

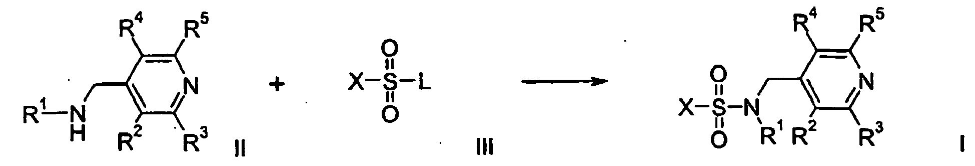

Diese Umsetzung erfolgt üblicherweise bei Temperaturen von-3u°C bis 120°C, vorzugsweise -10°C bis 100°C, in einem inerten organischen Lösungsmittel in Gegenwart einer Base [vgl. Lieb. Ann. Chem. 641 (1990)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, sowie Dimethylsulfoxid, Dimethylformamid und Dimethylacetamid, besonders bevorzugt Diisopropylether, Diethylether und Tetrahydrofuran. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Pyridin, Triethylamin und Kaliumcarbonat. Die Basen werden im allgemeinen in katalytischen Mengen eingesetzt, sie können aber auch äquimolar, im Überschuss oder gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuss bezogen auf III einzusetzen.

Die für die Herstellung der Verbindungen I benötigten Ausgangsstoffe sind kommerziell erhältlich oder in der Literatur bekannt [J. für praktische Chemie, S. 695 (1994); Heterocycles, S. 675 (1995); Tetrahedron, S. 12483 (1996); Chem. Pharm. Bull., S. 1927 (1973); J. Chem. Soc., S. 426 (1942); EP-A 983 982; Synthesis, S. 852 (1986)] oder können gemäß der zitierten Literatur hergestellt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 2 oder 4 Kohlenstoffatomen (wie vorstehend genannt), wobei in diesen Gruppen teilweise oder vollständig die Wasserstoffatome durch Halogenatome wie vorstehend genannt ersetzt sein können: insbesondere C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
Alkenyl: ungesättigte, geradkettige oder verzweigte Kohlenwasserstoffreste mit 2 bis 4, 6 oder 8 Kohlenstoffatomen und einer oder zwei Doppelbindungen in beliebiger Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl,-1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1 -Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Di-methyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-buten 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Halogenalkenyl: ungesättigte, geradkettige oder verzweigte Kohlenwasserstoffreste mit 2 bis 6 Kohlenstoffatomen und einer oder zwei Doppelbindungen in beliebiger Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
Alkinyl: geradkettige oder verzweigte Kohlenwasserstoffgruppen mit 2 bis 4, 6 oder 8 Kohlenstoffatomen und einer oder zwei Dreifachbindungen in beliebiger Position, z.B. C₂-C₆-Alkinyl wie Ethinyl, 1-Propinyl, 2-propionyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Cycloalkyl: mono- oder bicyclische, gesättigte Kohlenwasserstoffgruppen mit 3 bis 6 oder 8 Kohlenstoffringgliedem, z.B. C₃-C₈-Cycloalkyl wie Cyclopropyl, Cyclobutyl, Cyclopentyl, Cyclohexyl, Cycloheptyl und Cyclooctyl;
fünf- bis zehngliedriger gesättigter, partiell ungesättigter oder aromatischer Heterocyclus, enthaltend ein bis vier Heteroatome aus der Gruppe O, N oder S:
   - 5- oder 6-gliedriges Heterocyclyl, enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
   - 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Thienyl, 3-Thienyl, 3-Pyrazolyl, 4-pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;
Alkylen: divalente unverzweigte Ketten aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
Oxyalkylen: divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
Oxyalkylenoxy: divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;
Alkenylen: divalente unverzweigte Ketten aus 4 oder 6 CH-Gruppen, die durch konjugierte C=C-Doppelbindungen verbunden sind, z.B. CH=CH oder CH=CH-CH=CH.

In dem umfang der vorliegenden Erfindung sind die (R)- und (S)-Isomere und die Razemate von Verbindungen der Formel eingeschossen, die chirale Zentren aufweisen.

Im Hinblick auf ihre bestimmungsgemäße Verwendung der Sulfonamide der Formel I sind die folgenden Bedeutungen der Substituenten, und zwar jeweils für sich allein oder in Kombination, besonders bevorzugt

Verbindungen der Formel I, in der R¹ Wasserstoff, Methyl, Methoxy, Ethoxy, Allyl oder Propargyl, insbesondere Wasserstoff oder Methyl, stellen einen bevorzugten Gegenstand der Erfindung dar.

Gleichermaßen bevorzugt sind Verbindungen der Formel I, in der R², R³, R⁴ und R⁵ unabhängig voneinander Wasserstoff, Methyl, Ethyl, Fluor, Chlor, CF₃, OCF₃ oder OCHF₂ bedeuten.

Daneben sind auch Verbindungen der Formel I bevorzugt, in der mindestens eine, insbesondere eine oder zwei, Gruppen ausgewählt aus R², R³, R⁴ und R⁵ ungleich Wasserstoff sind.

Gleichfalls bevorzugt sind auch Verbindungen der Formel I, die durch zwei gleiche Gruppen ausgewählt aus R², R³, R⁴ und R⁵ substituiert sind.

Weitere bevorzugte Ausgestaltungen der Formel I sind jeweils für sich Verbindungen der Formeln I.1 bis I.6, wobei die Variablen wie für Formel I definiert sind:

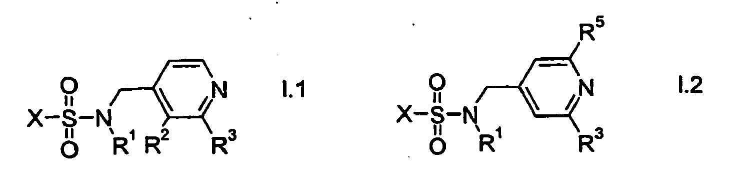

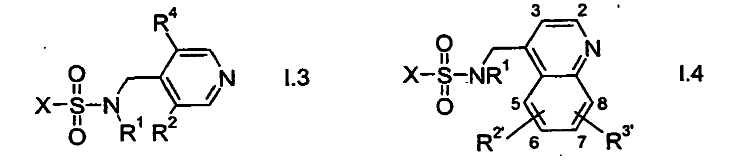

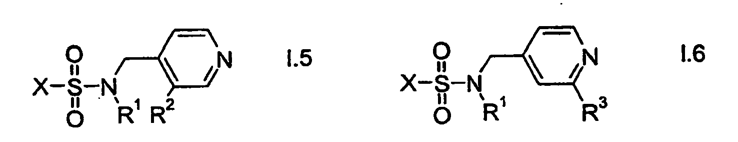

Verbindungen der Formel 1.4 sind bevorzugt, in denen die Gruppen R^{2'} und R^{3'} in 6,7-Stellung stehen.

Besonders bevorzugt sind Verbindungen der Formel I, in der X für einen Phenylring steht, der eine Gruppe R^{a} in para-Stellung trägt, diese Verbindungen entsprechen Formel IA:

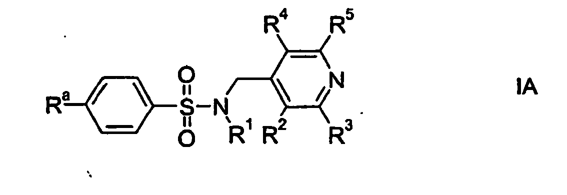

Verbindungen der Formel I sind besonders bevorzugt, in denen R^{a} folgende Bedeutungen hat C(R⁶)=NOR⁷, Methyl, Ethyl, n-Propyl, iso-Propyl, n-Butyl, iso-Butyl, sec. Butyl, tert. Butyl, Methoxy, Ethoxy, n-Propoxy, iso-Propoxy, n-Butoxy, iso-Butoxy, sec. Butoxy, tert. Butoxy, Chlormethyl, Dichlormethyl, Trichlormethyl, Fluormethyl, Difluormethyl, Trifluormethyl, Dichlorfluormethyl, Chlordifluormethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, Trichlormethoxy, Fluormethoxy, Difluormethoxy, Trifluormethoxy, Chlordifluomiethoxy, 2,2-Difluorethoxy, 2,2,2-Trifluorethoxy, 2,2,2-Trichlorethoxy und Pentafluorethoxy.

Eine besonders bevorzugte Ausgestaltung für R⁶ ist Methyl; R⁷ bedeutet bevorzugt Methyl, Ethyl, Allyl und Propargyl, wobei die Gruppen R⁷ halogeniert sein können.

Insbesondere sind im Hinblick auf ihre Verwendung die in den folgenden Tabellen zusammengestellten Verbindungen I bevorzugt Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der Formel IA, in denen R², R³, R⁴ und R⁵ Wasserstoff und die Kombination von R¹ und R^{a} für eine Verbindung jeweils einer Zeile A-1 bis A-14 der Tabelle A entspricht

### Tabelle 2

Verbindungen der Formel IA.1, in denen R² und R³ Methyl und die Kombination von R¹ und R^{a} für eine Verbindung jeweils einer Zeile der Tabelle A entspricht

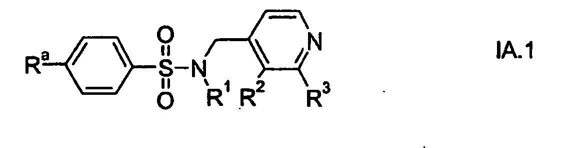

### Tabelle 3

Verbindungen der Formel IA.1, in denen R² und R³ Fluor und die Kombination von R¹ und R^{a} für eine Verbindung jeweils einer Zeile der Tabelle A entspricht

### Tabelle 4

Verbindungen der Formel IA.1, in denen R² und R³ Chlor und die Kombination von R¹ und R^{a} für eine Verbindung jeweils einer Zeile der Tabelle A entspricht

### Tabelle 5

Verbindungen der Formel IA.1, in denen R² und R³ Methoxy und die Kombination von R¹ und R^{a} für eine Verbindung jeweils einer Zeile der TabeHe A entspricht

### Tabelle 6

Verbindungen der Formel IA.1, in denen R² und R³ Trifluormethoxy und die Kombination von R¹ und R^{a} für eine Verbindung jeweils einer Zeile der Tabelle A entspricht

### Tabelle 7

Verbindungen der Formel IA.2, in denen R³ und R⁵ Methyl und die Kombination von R¹ und R^{a} für eine Verbindung jeweils einer Zeile der Tabelle A entspricht

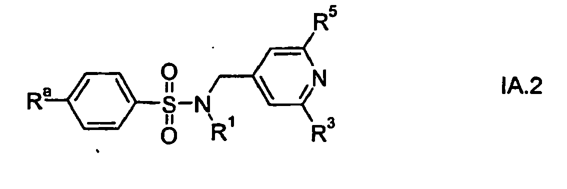

### Tabelle 8

Verbindungen der Formel IA.2, in denen R³ und R⁵ Fluor und die Kombination von R¹ und R^{a} für eine Verbindung jeweils einer Zeile der Tabelle A entspricht

### Tabelle 9

Verbindungen der Formel IA.2, in denen R³ und R⁵ Chlor und die Kombination von R¹ und R^{a} für eine Verbindung jeweils einer Zeile der Tabelle A entspricht

### Tabelle 10

Verbindungen der Formel IA.2, in denen R³ und R⁵ Methoxy und die Kombination von R¹ und R^{a} für eine Verbindung jeweils einer Zeile der Tabelle A entspricht

### Tabelle 11

Verbindungen der Formel IA-2, in denen R³ und R⁵ Trifluormethoxy und die Kombination von R¹ und R^{a} für eine Verbindung jeweils einer Zeile der Tabelle A entspricht

### Tabelle 12

Verbindungen der Formel IA.3, in denen R² und R⁴ Methyl und die Kombination von R¹ und R^{a} für eine Verbindung jeweils einer Zeile der Tabelle A entspricht

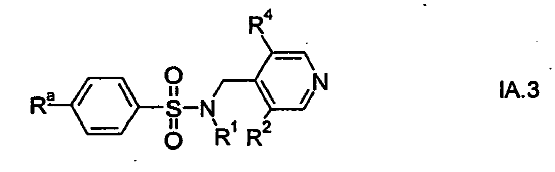

### Tabelle 13

Verbindungen der Formel IA.3, in denen R² und R⁴ Fluor und die Kombination von R¹ und R^{a} für eine Verbindung jeweils einer Zeile der Tabelle A entspricht

### Tabelle 14

Verbindungen der Formel IA.3, in denen R² und R⁴ Chlor und die Kombination von R¹ und R^{a} für eine Verbindung jeweils einer Zeile der Tabelle A entspricht

### Tabelle 15

Verbindungen der Formel IA.3, in denen R² und R⁴ Methoxy und die Kombination von R¹ und R^{a} für eine Verbindung jeweils einer Zeile der Tabelle A entspricht

### Tabelle 16

Verbindungen der Formel IA.3, in denen R² und R⁴ Trifluormethoxy und die Kombination von R¹ und R^{a} für eine Verbindung jeweils einer Zeile der Tabelle A entspricht

### Tabelle 17

Verbindungen der Formel IA.4', in denen R² und R³ Wasserstoff und die Kombination von R¹ und R^{a} für eine Verbindung jeweils einer Zeile der Tabelle A entspricht

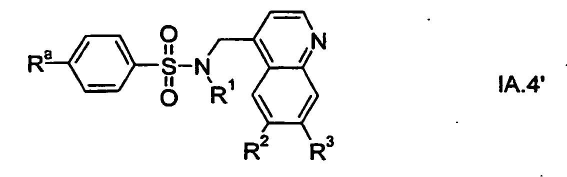

### Tabelle 18

Verbindungen der Formel IA.4', in denen R² und R³ Methyl und die Kombination von R¹ und R^{a} für eine Verbindung jeweils einer Zeile der Tabelle A entspricht

### Tabelle 19

Verbindungen der Formel IA.4', in denen R² und R³ Fluor und die Kombination von R¹ und R^{a} für eine Verbindung jeweils einer Zeile der Tabelle A entspricht

### Tabelle 20

Verbindungen der Formel IA.4', in denen R² und R³ Chlor und die Kombination von R¹ und R^{a} für eine Verbindung jeweils einer Zeile der Tabelle A entspricht

### Tabelle 21

Verbindungen der Formel IA-4', in denen R² und R³ Methoxy und die Kombination von R¹ und R^{a} für eine Verbindung jeweils einer Zeile der Tabelle A entspricht

### Tabelle 22

Verbindungen der Formel IA.4', in denen R² und R³ Trifluormethoxy und die Kombination von R¹ und R^{a} für eine Verbindung jeweils einer Zeile der Tabelle A entspricht

### Tabelle 23

Verbindungen der Formel IA.5, in denen R² Methyl und die Kombination von R¹ und R^{a} für eine Verbindung jeweils einer Zeile der Tabelle A entspricht

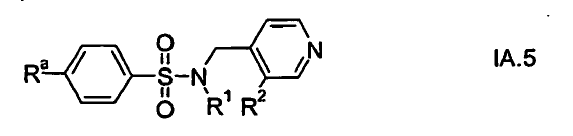

### Tabelle 24

Verbindungen der Formel IA,5, in denen R² Fluor und die Kombination von R¹ und R^{a} für eine Verbindung jeweils einer Zeile der Tabelle A entspricht

### Tabelle 25

Verbindungen der Formel IA.5, in denen R² Chlor und die Kombination von R¹ und R^{a} für eine Verbindung jeweils einer Zeile der Tabelle A entspricht

### Tabelle 26

Verbindungen der Formel IA.5, in denen R² Methoxy und die Kombination von R¹ und R^{a} für eine Verbindung jeweils einer Zeile der Tabelle A entspricht

### Tabelle 27

Verbindungen der Formel IA.5, in denen R² Trifluormethoxy und die Kombination von R¹ und R^{a} für eine Verbindung jeweils einer Zeile der Tabelle A entspricht

### Tabelle 28

Verbindungen der Formel IA.6, in denen R³ Methyl und die Kombination von R¹ und R^{a} für eine Verbindung jeweils einer Zeile der Tabelle A entspricht

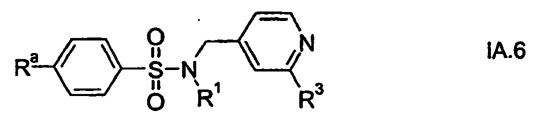

### Tabelle 29

Verbindungen der Formel IA.6, in denen R³ Fluor und die Kombination von R¹ und R^{a} für eine Verbindung jeweils einer Zeile der Tabelle A entspricht

### Tabelle 30

Verbindungen der Formel IA.6, in denen R³ Chlor und die Kombination von R¹ und R^{a} für eine Verbindung jeweils einer Zeile der Tabelle A entspricht

### Tabelle 31

Verbindungen der Formel IA.6, in denen R³ Methoxy und die Kombination von R¹ und R^{a} für eine Verbindung jeweils einer Zeile der Tabelle A entspricht

### Tabelle 32

Verbindungen der Formel IA.6, in denen R³ Trifluormethoxy und die Kombination von R¹ und R^{a} für eine Verbindung jeweils einer Zeile der Tabelle A entspricht

**Tabelle A**

| **Nr.** | **R¹** | **R^{a}** |
|---|---|---|
| A-1 | H | C(CH₃)=NOCH₃ |
| A-2 | CH₃ | C(CH₃)=NOCH₃ |
| A-3 | H | C(CH₃)=NOCH₂CH₃ |
| A-4 | CH₃ | C(CH₃)=NOCH₂CH₃ |
| A-5 | H | C(CH₃)=NOCH₂CH=CH₂ |
| A-6 | CH₃ | C(CH₃)=NOCH₂CH=CH₂ |
| A-7 | H | C(CH₃)=NOCH₂C≡CH |
| A-8 | CH₃ | C(CH₃)=NOCH₂C≡CH |
| A-9 | H | C(CH₃)=NOCH₂CCl≡CH₂ |
| A-10 | CH₃ | C(CH₃)=NOCH₂CCl=CH₂ |
| A-11 | H | H |
| A-12 | CH₃ | H |
| A-13 | H | CH₃ |
| A-14 | CH₃ | CH₃ |
| A-15 | H | CH₂CH₃ |
| A-16 | CH₃ | CH₂CH₃ |
| A-17 | H | CH₂CH₂CH₃ |
| A-18 | CH₃ | CH₂CH₂CH₃ |
| A-19 | H | CH(CH₃)₂ |
| A-20 | CH₃ | CH(CH₃)₂ |
| A-21 | H | CH₂CH₂CH₂CH₃ |
| A-22 | CH₃ | CH₂CH₂CH₂CH₃ |
| A-23 | H | CH(CH₃)CH₂CH₃ |
| A-24 | CH₃ | CH(CH₃)CH₂CH₃ |
| A-25 | H | CH₂CH(CH₃)₂ |
| A-26 | CH₃ | CH₂CH(CH₃)₂ |
| A-27 | H | C(CH₃)₃ |
| A-28 | CH₃ | C(CH₃)₃ |
| A-29 | H | OCH₃ |
| A-30 | CH₃ | OCH₃ |
| A-31 | H | OCH₂CH₃ |
| A-32 | CH₃ | OCH₂CH₃ |
| A-33 | H | OCH₂CH₂CH₃ |
| A-34 | CH₃ | OCH₂CH₂CH₃ |
| A-35 | H | OCH(CH₃)₂ |
| A-36 | CH₃ | OCH(CH₃)₂ |
| A-37 | H | OCH₂CH₂CH₂CH₃ |
| A-38 | CH₃ | OCH₂CH₂CH₂CH₃ |
| A-39 | H | OCH(CH₃)CH₂CH₃ |
| A-40 | CH₃ | OCH(CH₃)CH₂CH₃ |
| A-41 | H | OCH₂CH(CH₃)₂ |
| A-42 | CH₃ | OCH₂CH(CH₃)₂ |
| A-43 | H | OC(CH₃)₃ |
| A-44 | CH₃ | OC(CH₃)₃ |
| A-45 | H | CCl₃ |
| A-46 | CH₃ | CCl₃ |
| A-47 | H | CHF₂ |
| A-48 | CH₃ | CHF₂ |
| A-49 | H | CF₃ |
| A-50 | CH₃ | CF₃ |
| A-51 | H | CHClF |
| A-52 | CH₃ | CHClF |
| A-53 | H | CH₂CHF₂ |
| A-54 | CH₃ | CH₂CHF₂ |
| A-55 | H | CH₂CF₃ |
| A-56 | CH₃ | CH₂CF₃ |
| A-57 | H | CF₂CF₃ |
| A-58 | CH₃ | CF₂CF₃ |
| A-59 | H | OCHCl₂ |
| A-60 | CH₃ | OCHCl₂ |
| A-61 | H | OCCl₃ |
| A-62 | CH₃ | OCCl₃ |
| A-63 | H | OCH₂F |
| A-64 | CH₃ | OCH₂F |
| A-65 | H | OCHF₂ |
| A-66 | CH₃ | OCHF₂ |
| A-67 | H | OCF₃ |
| A-68 | CH₃ | OCF₃ |
| A-69 | H | OCH₂CHF₂ |
| A-70 | CH₃ | OCH₂CHF₂ |
| A-71 | H | OCH₂CF₃ |
| A-72 | CH₃ | OCH₂CF₃ |
| A-73 | H | OCH₂CHClF |
| A-74 | CH₃ | OCH₂CHClF |
| A-75 | H | OCH₂CCl₃ |
| A-76 | CH₃ | OCH₂CCl₃ |
| A-77 | H | OCF₂CF₃ |
| A-78 | CH₃ | OCF₂CF₃ |
| A-79 | H | C₆H₅ |
| A-80 | CH₃ | C₆H₅ |
| A-81 | H | OC₆H₅ |
| A-82 | CH₃ | OC₆H₅ |

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und *Basidiomyceten.* Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alfernaria*-Arten an Gemüse und Obst,
- *Bipolaris-* und *Drechslera-A*rten an Getreide, Reis und Rasen,
- *Blumeria graminis* (echter Mehltau) an Getreide,
- *Botrytis cinerea* (Grauschimmel) an Erdbeeren, Gemüse, Zierpflanzen und Reben,
- *Erysiphe cichoracearum* und *Sphaerotheca fuliginea* an Kürbisgewächsen,
- *Fusarium-* und *Verticillium-Arten* an verschiedenen Pflanzen,
- Mycosphaerella-Arten an Getreide, Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Rhizoctonia*-Arten an Baumwolle, Reis und Rasen,
- *Septoria tritici* und Stagonospora *nodorum* an Weizen,
- *Uncinula necator* an Reben,
- *Ustilago*-Arten an Getreide und Zuckerrohr, sowie
- *Venturia*-Arten (Schorf) an Äpfeln und Birnen.

Die Verbindungen I eignen sich außerdem zur Bekämpfung von Schadpilzen *wie Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin; Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht. Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind: 1. Produkte zur Verdünnung in Wasser

### A Wasserlösliche Konzentrate (SL)

10 Gew.-Teile einer erfindungsgemäßen Verbindung werden in Wasser oder einem wasserlöslichen Lösungsmittel gelost. Alternativ werden Netzmittel oder andere Hilfsmittel zugefügt. Bei der Verdünnung in Wasser löst sich der Wirkstoff.

### B Dispergierbare Konzentrate (DC)

20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.

### C Emulgierbare Konzentrate (EC)

15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.

### D Emulsionen (EW, EO)

40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.

### E Suspensionen (SC, OD)

20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.

### F Wasserdispergierbare und wasserlösliche Granulate (WG, SG)

50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.

### G Wasserdispergierbare und wasserlösliche Pulver (WP, SP)

75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

### H Stäube (DP)

5 Gew.Teile einer erfindungsgemäßen Verbindung werden fein gemahlen und mit 95 % feinteiligem Kaolin innig vermischt. Man erhält dadurch ein Stäubemittel.

### I Granulate (GR, FG, GG, MG)

0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.

### J ULV- Lösungen (UL)

10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubmitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyrodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Epoxiconazol, Fenbuconazol, Fluquiconazol, Flusilazol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel
- Sonstige Fungizide wie Acibenzotar-S-methyl, Benthiavalicarb, Carpropamid, Chlorothalonil, Cyflufenamid, Cymoxanil, Dazomet, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phthalid, Toloclofos-methyl, Quintozene, Zoxamid
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1: Herstellung von 4-Acetyl-N-pyridin-4-yl-methyl-phenylsulfonamid

Einer Lösung von 10 g (45,7 mmol) 4-Acetylsulfonsäurechlorid in 150 ml Diethylether wurden 1 ml Pyridin bei -10 °C die Lösung von 4,95 g (45,7 mmol) 4-(Aminomethyl)-pyridin (4-Picotylamin) in 10 ml Diethylether zugetropft; dann wurde die Lösung bei 20-25°C etwa 18 Std. gerührt. Das Produkt wurde abgesaugt, der Rückstand mit verd. NaHCO₃-Lösung und Wasser gewaschen, dann getrocknet Man erhielt 5,2 g der Titelverbindung vom Fp: 162-167 °C.

### Beispiel 2: Herstellung von 4-(1-Ethoxyimino-ethyl)-N-pyridin-4-yl-methyl-phenyl-sulfonamid

Eine Lösung von 0,4 g (1,3 mmol) der Verbindung aus Beispiel 1 in 20 ml Methanol wurde mit 0,42 g einer 40%igen wässr. O-Ethylhydroxylamin-Lösung versetzt. Mit 10%iger Salzsäure wurde auf pH 4 angesäuert; dann wurde die Lösung bei 20-25°C etwa 18 Std. gerührt. Die Reaktionslösung wurde auf Wasser gegeben und mit NaHCO₃ auf pH 8 gestellt. Anschließend wurde mit Methyl-tert.Butylether (MtBE) extrahiert und die vereinigten organischen Phasen mit Wasser gewaschen und getrocknet. Nach Entfernen des Lösungsmittels erhielt man 0,4 g der Titelverbindung als dickflüssiges Öl.
¹H-NMR (δ, CDCl₃,): 8,5 (d, 2H); 7,5 (m, 4H); 7,1 (d, 2H); 5,0 (t,1H); 4,25 (q, 2H); 4,1 (d, 2H); 2,25 (s, 3H); 1,3 (t, 3H).

### Beispiel 3: Herstellung von 4-(1-Ethoxyimino-ethyl)-N-methyl-N-pyridin-4-yl-methyl-phenylsulfonamid

Eine Aufschlämmung von 0,04 g (1,32 mmol) NaH (95%ig) in 50 ml Dimethylformamid (DMF) wurde mit 0,4 g (1,2 mmol) der Verbindung aus Beispiel 2 versetzt, dann 10 min bei 20-25°C gerührt. Anschließend wurde eine Lösung von 0,17 g (1,2 mmol) Jodmethan in 10 ml DMF zugetropft; die vereinigte Reaktionslösung wurde bei 20-25°C etwa 18 Std. gerührt, auf Wasser gegeben, dann mit MtBE extrahiert. Die den organischen Phasen wurden mit Wasser gewaschen, dann getrocknet. Nach Entfernen des Lösungsmittels erhielt man 0,3 g der Titelverbindung als dickflüssiges Öl.
¹H-NMR (δ,CDCl₃,): 8,6 (d, 2H); 7,8 (m, 4H); 7,25 (d, 2H); 4,25 (q, 2H); 4,1 (d, 2H); 2,6 (s, 3H); 2,25 (s, 3H); 1,25 (t, 3H).

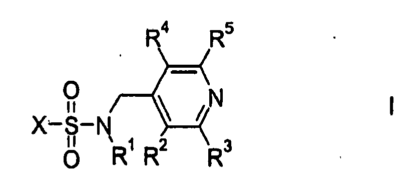

**Tabelle I**

| **Nr.** | **X** | **R¹** | **R²** | **R³** | **R⁴** | **R⁵** | **phys. Daten (Fp.[°C]; ¹H-NMR δ [ppm]; MS m/e [M+H⁺])** |
|---|---|---|---|---|---|---|---|
| I-1 | 4-(C[CH₃]=NOCH₃)-C₆H₄ | H | H | H | H | H | 4,2 (2H); 4,0 (3H); 4,2 (2H); 4,0 (3H); |
| I-2 | 4-(C[CH₃]=NOCH₂CH₃)-C₈H₄ | H | H | H | H | H | 4,25 (2H); 4,2 (2H); 2,25 (3H); 1,3 (3H) |
| I-3 | 4-(C[CH₃]=NOCH₂CH=CH₂)-C₈H₄ | H | H | H | H | H | 5,2 (2H); 4,7 (2H); 4,05 (2H); 2,25 (3H) |
| I-4 | 4-(C[CH₃]=NOCH(CH₃)₂)-C₈H₄ | H | H | H | H | H | 4,45 (1H); 4,2 (2H); 2,25 (3H); 1,3 (6H) |
| I-5 | 4-(C[CH₃]=NOCH₂C=CH)-C₈H₄ | H | H | H | H | H | 4,8 (2H); 4,2 (2H); 2,5 (1H); 2,3 (3H) |
| I-6 | 4-(C[CH₃]=NO(CH₂)₅CH₃)-C₆H₄ | H | H | H | H | H | 4,2 (2H); 4,1 (2H); 2,25 (3H); 0,9 (3H) |
| I-7 | 4-(C[CH₃]=NOCH₂C₆H₅)-C₆H₄ | H | H | H | H | H | 2,25 4,2.5 (2H); 2,25 (3H) |
| I-8 | 4-(C[CH₃]=NOCH₃)-C₆H₄ | CH₃ | H | H | H | H | 4,2 (2H); 4,05 (3H); 2,65 (3H); 2,25 (3H) |
| I-9 | 4-(C[CH₃]=NOCH₂CH₃)-C₆H₄ | CH₃ | H | H | H | H | 2,7 (2H); 1,35 (3H) 2,7 (3H); 1,35 (3H) |
| I-10 | 4-(C[CH₃]=NOCH₂CH₃)-C₈H₄ | CH₂CH₃ | H | H | H | H | 4,3 (2H); 4,2 (2H); 3,2 (2H); 2,2 (3H); 1,3 (3H); 0,9 (3H) |
| I-11 | 4-(C[CH₃]=NOCH₂CH₃)-C₆H₄ | CH₂C ≡CH | H | H | H | H | 4,35 (2H); 4,25 (2H); 4,0 (2H); 2,25 (1H) |
| I-12 | 4-(C[CH₃]=NOCH₂CH₃)-C₆H₄ | C₆H₅CH₂ | H | H | H | H | 4,3 (2H); 4,25 (2H); 4,2 (2H); 2,25 (3H) |
| I-13 | 4-(C[CH₃]=NOCH₂CH₃)-C₆H₄ | CH₂CH=CH₂ | H | H | H | H | 5,5 (1H); 5,0 (2H); 4,3 (2H); 3,75 (2H) |
| I-14 | 4-COOCH₃-C₈H₄ | H | -CH=CH-CH=CH- | | H | H | 8,45 (1H); 8,0 (1H); 7,4 (1H); 4,5 (2H) |
| I-15 | 4-CH₃-C₆H₄ | H | -CH=CH-CH=CH- | | H | H | 8,1 (1H), 7,3 (1H); 4,65 (2H); 1,3 (9H) |
| I-16 | 4-OCH₃-C₆H₄ | H | -CH=CH-CH=CH- | | H | H | 8,1 (1H); 4,6 (1H); 3,0 (1H); 1,3 (6H) |
| I-17 | 4-Cl-C₆H₄ | H | -CH=CH-CH=CH- | | H | H | m/e 333 |
| I-18 | 2,5-Cl₂-C₆H₃ | H | -CH=CH-CH=CH- | | H | H | m/e 367 |
| I-19 | 1-Naphthyl | H | -CH=CH-CH=CH- | | H | H | m/e 349 |
| I-20 | 2-CH₃-6-CF₃-C₆H₃ | H | -CH=CH-CH=CH- | | H | H | m/e 381 |
| I-21 | 2-Cl-5-OCH₃-C₆H₃ | H | -CH=CH-CH=CH- | | H | H | m/e 363 |
| I-22 | 2,4-Cl₂-C₆H₃ | H | -CH=CH-CH=CH- | | H | H | m/e 367 |
| I-23 | 4-CN-C₆H₄ | H | -CH=CH-CH=CH- | | H | H | m/e 324 |
| I-24 | 2,6-C1₂-C₆H₃ | H | -CH=CH-CH=CH- | | H | H | m/e 367 |
| I-25 | 2-Br-C₆H₄ | H | -CH=CH-CH=CH- | | H | H | m/e 379 |
| I-26 | 2,3-Cl₂-C₆H₃ | H | -CH=CH-CH=CH- | | H | H | m/e 367 |
| I-27 | 3,4-(OCH₃)₂-C₈H₃ | H | -CH=CH-CH=CH- | | H | H | m/e 358 |
| I-28 | 2-CH₃-6-Cl-C₆H₃ | H | -CH=CH-CH=CH- | | H | H | m/e 347 |
| I-29 | 2-Cl-C₆H₄ | H | -CH=CH-CH=CH- | | H | H | m/e 333 |
| I-30 | C₆H₅ | H | -CH=CH-CH=CH- | | H | H | 8,45 (1H); 8,0 (1H); 7,4 (1H); 4,5 (2H) |
| I-31 | 4-C(CH₃)₃-C₆H₄ | H | -CH=CH-CH=CH- | | H | H | 8,1 (1H); 7,3 (1H); 4,65 (2H); 1,3 (9H) |
| I-32 | 4-CH(CH₃)₂-C₆H₄ | H | -CH=CH-CH=CH- | | H | H | 3,0 (1H); 4,6 (1H); 8,75 (1H); 8,0 (1H); |
| I-33 | 4-0CF₃-C₆H₄ | H | -CH=CH-CH=CH- | | H | H | 5,8 (1H); 4,7 (2H) |
| I-34 | 4-OCF₂CHF₂-C₆H₄ | H | -CH=CH-CH=CH- | | H | H | 8,8 (1H); 4,7 (2H) |
| I-35 | 4-COCH₃-C₆H₄ | H | -CH=CH-CH=CH- | | H | H | 4,6 (2H);2,6 (3H) |
| I-36 | 4-(C[CH₃]=NOCH₃)-C₆H₄ | H | -CH=CH-CH=CH- | | H | H | 5,2 (NH); 4,6 (2H); 4,0 (3H); 2,2 (3H) |
| I-37 | 4-(C[CH₃]=NOCH₂CH₃)-C₆H₄ | H | -CH=CH-CH=CH- | | H | H | 5,0 (NH); 4,6 (2H); 4,3 (3H); 2,3 (3H); 1,35 (3H) |
| I-38 | 4-(C[CH₃]=NOCH(CH₃)₂)-C₆H₄ | H | -CH=CH-CH=CH- | | H | H | 5,1 (NH); 4,6 (2H); 4,5 (3H); 2,25 (3H); 1,3 (6H) |
| I-39 | 4-(C[CH₃]=NOCH₂CH=CH₂)-C₆H₄ | H | -CH=CH-CH=CH- | | H | H | 5,3 (NH); 5,2 (2H); 4,75 (2H); 4,6 (2H); 2,35 (3H) |
| I-40 | 4-(C[CH₃]=NOCH₂C≡CH)-C₆H₄ | H | -CH=CH-CH=CH- | | H | H | 5,1 (NH); 4,8 (2H); 4,6 (2H); 2,5 (2H); 2,3 (3H) |
| I-41 | 4-(C[CH₃]=NO(CH₂)₅CH₃)-C₆H₄ | H | -CH=CH-CH=CH- | | H | H | 5,2 (NH); 4,6 (2H); 4,2 (2H); 2,25 (2H); 0,9 (3H) |
| I-42 | 4-(C[CH₃]=NOCH₂C₆H₅)-C₆H₄ | H | -CH=CH-CH=CH- | | H | H | 5,3 (2H); 5,2 (NH); 4,8 (2H); 2,3 (3H) |
| I-43 | C₆H₅ | CH₃ | -CH=CH-CH=CH- | | H | H | 4,6 (2H); 2,7 (3H) |
| I-44 | 5-Cl-thlophen-2-yl | H | H | H | H | H | mle 339 |
| I-45 | Thiophen-2-yl | H | H | H | H | H | m/e 305 |
| I-46 | 5-([4-C(CH₃)₃]-C₆H₄)-thiophen-2-yl | H | H | H | H | H | 166 |
| I-47 | 5-Br-thiophen-2-yl | H | H | H | H | H | 148 |
| I-48 | 4-NO₂-5-Cl-thlophen-2-yl | H | H | H | H | H | 182 |
| I-49 | 5-Cl-1,3-(CH₃)₂-1H-pyrazol-4-yl | H | H | H | H | H | 147 |
| I-50 | 3-Br-2,5-Cl₂-thiophen-4-yl | H | H | H | H | H | 106 |
| I-51 | 1-CH₃-1H-imidazol-4-yl | H | H | H | H | H | 125 |
| I-52 | 5-(4-tert-Butylphenyl)-thiophen-2-yl | H | H | H | H | H | 166 |
| I-53 | 5-Chlor-1,3-dimethyl-1H-pyrazol-4-yl | H | H | H | H | H | 147 |
| I-54 | 5-Brom-thiophen-2-yl | H | H | H | H | H | 148 |
| I-55 | 5-Chlor-4-nitro-thiophen-2-yl | H | H | H | H | H | 182 |
| I-56 | 1-Methyl-1H-imidazol-4-yl | H | H | H | H | H | 125 |
| I-57 | 4-Brom-2,5-dichlor-thlophen-3-yl | H | H | H | H | H | 106 |
| I-58 | 5-Biphenyl-4-yl-thiophen-2-yl | H | H | H | H | H | 204 |
| I-59 | 5-(4-Trifluoromethoxy-phenyl)-thio-phen-2-yl | H | H | H | H | H | 138 |
| I-60 | 5-(4-Propyl-phenyl)-thiophen-2-yl | H | H | H | H | H | 144 |
| I-61 | 5-(4-Ethyl-phenyl)-thiophen-2-yl | H | H | H | H | H | 144 |
| I-62 | 5-(3-Trifluoromethyl-phenyl)-thio-phen-yl | H | H | H | H | H | 130 |
| I-63 | 5-(4-Chloro-phenyl)-thiophen-2-yl | H | H | H | H | H | 176 |
| I-64 | 5-(4-Trifluoromethyl-phenyl)-thio-phen-yl | H | H | H | H | H | 165 |
| I-65 | 5-(4-Methoxy-phenyl)-thiophen-2-yl | H | H | H | H | H | 175 |
| I-66 | 5-(4-Trifluoromethyl-phenyl)-thio-phen-2-yl | Propargyl | H | H | H | H | 116 |
| I-67 | 5-(4-Trifluoromethyl-phenyl)-thio-phen-2-yl | n-Propyl | H | H | H | H | 97 |
| I-68 | 5-(4-Trifluoromethyl-phenyl)-thio-phen-2-yl | Methyl | H | H | H | H | 101 |
| I-69 | 5-(4-Isopropyl-phenyl)-thiophen-2-yl | H | H | H | H | H | 125 |
| I-70 | 5-Brom-thlophen-2-yl | H | -CH=CH-CH=CH- | | H | H | 137 |
| I-71 | 5-(4-Trifluoromethyl-phenyl)-thio-phen-2-yl | H | -CH=CH-CH=CH- | | H | H | 180 |
| I-72 | 4-tert.Butyl-phenyl | Methyl | -CH=CH-CH=CH- | | H | H | 145-148 |

### Beispiele für die Wirkung gegen Schadpilze

Die fungizide Wirkung der Verbindungen der Formel 1 ließ sich durch die folgenden Versuche zeigen:

Die Wirkstoffe wurden getrennt oder gemeinsam als eine Stammlösung aufbereitet mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf der Basis ethoxylierter Alkylphenole) zugesetzt und entsprechend der gewünschten Konzentration mit wasser verdünnt

### Anwendungsbeispiel 1 - Wirksamkeit gegen die Dürrfleckenkrankheit der Tomate verursacht durch Alternaria solani

Blätter von Topfpflanzen der Sorte "Goldene Prinzessin" wurden mit einer wässriger Suspension in der unten angegebenen Wirkstoffkonzentration bis zur Tropfnässe besprüht Am folgenden Tag wurden die Blätter mit einer wässrigen Sporenaufschwemmung von *Alternaria solani* in 2 % Biomalzlösung mit einer Dichte von 0,17 x 10⁶ Sporen/ml infiziert Anschließend wurden die Pflanzen in einer wasserdampf-gesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, dass der Befall visuell in % ermittelt werden konnte.

In diesem Test zeigten die mit 250 ppm der Wirkstoffe I-2 bis I-6, I-14, I-15, I-17, I-30 bis I-34, I-36 bis I-42, bzw. I-45 behandelten Pflanzen nicht über 30 % Befall, während die unbehandelten Pflanzen zu 100 % befallen waren.

### Anwendungsbeispiel 2 - Wirksamkeit gegen Mehltau an Gurkenblättem verursacht durch Sphaerotheca fuliginea bei protektiver Anwendung

Blätter von in Töpfen gewachsenen Gurkenkeimlingen der Sorte "Chinesische Schlange" wurden im Keimblattstadium mit wässriger Suspension in der unten angegebenen Wirkstoffkonzentration bis zur Tropfnässe besprüht 20 Stunden nach dem Antrocknen des Spritzbelages wurden die Pflanzen mit einer wässrigen Sporensuspension des Gurkenmehltaus *(Sphaerotheca fuliginea)* inokuliert. Anschließend wurden die Pflanzen im Gewächshaus bei Temperaturen zwischen 20 und 24°C und 60 bis 80 % relativer Luftfeuchtigkeit für 7 Tage kultiviert Dann wurde das Ausmaß der Mehltauentwicklung visuell in %-Befall der Keimblattfläche ermittelt.

In diesem Test zeigten die mit 250 ppm der Wirkstoffe I-1 bis I-5, I-15, I-18, I-30, I-32, I-33, I-34, I-37 bis I-40, I-42, bzw. I-45 behandelten Pflanzen nicht über 30 % Befall, während die unbehandelten Pflanzen zu 100 % befallen waren.

## Patentansprüche

1. Sulfonamide der Formel I 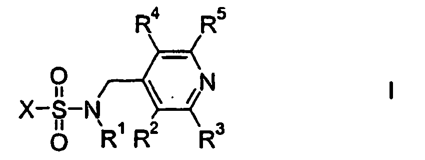 in der die Substituenten folgende Bedeutung haben:
R' Wasserstoff. C₁-C₄-Alkyl, C₁-C₄-Alkoxy, C₂-C₄-Alkenyl, C₂-C₄-Alkinyl oder Benzyl;
R², R³, R⁴, R⁵ unabhängig voneinander Wasserstoff, Halogen, C₁-C₄-Alkyl, C₂-C₄-Alkoxy oder C₁-Halogenmethyl;
R² und R³ können auch gemeinsam einen Phenyl-, Cyclopentyl- oder Cyclohexyl-
ring bilden, wobei diese Ringe zwei Gruppen R^{2,} und R^{3,} tragen können,
R^{2,}, R^{3,} unabhängig voneinander Wasserstoff, Halogen, C₁-C₄-Alkyl, C₁-C₄-Alkoxy oder C₁-Halogenmethyl;
im Fall a), wenn R², R³, R⁴ und R⁵ Wasserstoff bedeuten:
X Phenyl, welches durch eine Gruppe -C(R⁶)=NOR⁷, mit
R⁶ C₁-C₄-Alkyl und
R⁷ C₁-C₈-Alkyl, Benzyl, C₂-C₄-Alkenyl, C₁-C₄-Halogenalkyl, C₂-C₄- Halogenalkenyl, C₂-C₄-Alkinyl oder C₂-C₄-Halogenalkinyl substituiert ist; und
im Fall b), wenn mindestens eine der Gruppen R², R³, R⁴ und R⁵ nicht Wasserstoff bedeutet
X Phenyl, Naphthyl oder ein fünf- oder sechsgliedriger über ein C-Atom ge- bundener gesättigter, partiell ungesättigter oder aromatischer Heterocyclus, enthaltend ein bis vier Heteroatome aus der Gruppe O, N oder S, wobei X eine bis vier Gruppen R^{a} tragen kann:
R^{a} Halogen, Cyano, Nitro, C₁-C₈-Alkyl, C₁-C₈-Alkoxy, C₁-C₈-Halogen- alkoxy, C₁-C₄-Alkylcarbonyl, C₁-C₄-Alkoxycarbonyl, -C(R⁶)=NOR⁷, C₁-C₄-Alkylaminocarbonyl, Di-(C₁-C₄-alkyl)aminocarbonyl oder Phenyl oder Phenoxy, wobei die Ringe eine bis drei Gruppen R^{b} tra- gen können:
R^{b} Halogen, C₁-C₄-Alkyl, C₁-C₄-Alkoxy, C₁-Halogenalkyl oder C₁-Halogenalkoxy;
R^{a} oder R^{b} kann auch eine C₃-C₄-A!ky)en- oder eine C₄-Alkenylen- gruppe bedeuten, die mit zwei benachbarten Ringgliedern des Phenylringes, an den sie gebunden ist, einen Ring bilden, der durch eine oder mehrere der voranstehend genannten Gruppen R^{a} oder R^{b} substituiert sein kann.

2. Verbindungen der Formel I gemäß Anspruch 1, in der R², R³, R⁴ und R⁵ unabhängig voneinander Wasserstoff, Methyl, Fluor, Chlor, CF₃, OCF₃ oder OCHF₂ bedeuten.

3. Verbindungen der Formel I gemäß Anspruch 1 oder 2, in der R¹ Wasserstoff, Methyl, Methoxy, Ethoxy, Allyl oder Propargyl bedeutet.

4. Verbindungen der Formel I gemäß einem der Ansprüche 1 bis 3, in der X für einen Phenylring steht, der in para-Stellung substituiert ist

5. Verbindungen der Formel I gemäß einem der Ansprüche 1 bis 3, in der X für einen aromatischen Heterocyclus steht.

6. Verfahren zur Herstellung der Verbindungen der Formel I gemäß einem der Ansprüche 1 bis 5, durch Umsetzung von Pyridinderivaten der Formel II, in der die Variablen gemäß Anspruch 1 definiert sind, 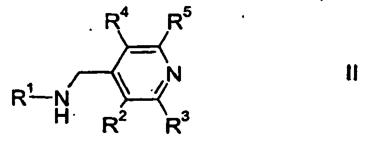 mit Sulfonsäurechloriden der Formel III, in der X die Bedeutung gemäß Anspruch 1 hat, 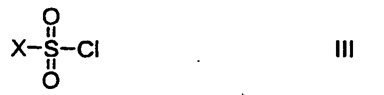 unter basischen Bedingungen.

7. Zur Bekämpfung von Schadpilzen geeignetes Mittel, enthaltend einen festen oder flüssigen Trägerstoff und eine Verbindung der Formel I gemäß Anspruch 1.

8. Verfahren zur Bekämpfung von pflanzenpathogenen Schadpilzen, **dadurch gekennzeichnet, dass** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

9. Pyridinderivate der Formel II gemäß Anspruch 6, in der die Substituenten folgende Bedeutung haben:
R¹ Wasserstoff oder Methyl;
R⁴, R⁵ Wasserstoff; und
R² = R³ beide Methyl, Fluor, Chlor, Methoxy oder Trifluormethoxy.

10. Pyridinderivate der Formel II gemäß Anspruch 6, in der die Substituenten folgende Bedeutung haben:
R¹ Wasserstoff oder Methyl;
R², R⁴ Wasserstoff; und
R³ = R⁵ beide Fluor, Methoxy oder Trifluormethoxy.

11. Pyridinderivate der Formel II gemäß Anspruch 6, in der die Substituenten folgende Bedeutung haben:
R¹ Wasserstoff oder Methyl;
R³, R⁵ Wasserstoff; und
R² = R⁴ beide Methyl, Fluor, Methoxy oder Trifluormethoxy.

12. Pyridinderivate der Formel II nach einem der Anspruch 9 bis 11, in der R¹ Wasserstoff bedeutet.

## Claims

1. A sulfonamide of the formula I 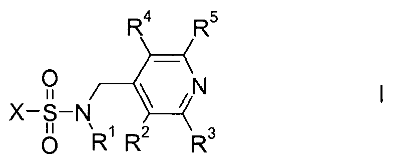 where the substituents are as follows:
R¹ is hydrogen, C₁-C₄-alkyl, C₁-C₄-alkoxy, C₂-C₄-alkenyl, C₂-C₄-alkynyl or ben- zyl;
R², R³, R⁴, R⁵ independently of one another are hydrogen, halogen, C₁-C₄- alkyl, C₁-C₄-alkoxy or C₁-halomethyl;
R² and R³ together may also form a phenyl, cyclopentyl or cyclohexyl ring, it be-
ing possible for these rings to carry two groups R², and R^{3,},
R², R³, independently of one another are hydrogen, halogen, C₁-C₄-alkyl, C₁-C₄-alkoxy or C₁-halomethyl;
in case a), if R², R³, R⁴ and R⁵ are hydrogen:
X is phenyl substituted by a group -C(R⁶)=NOR⁷, where
R⁶ is C₁-C₄-alkyl and
R⁷ is C₁-C₈-alkyl, benzyl, C₂-C₄-alkenyl, C₁-C₄-haloalkyl, C₂-C₄- haloalkenyl, C₂-C₄-alkynyl or C₂-C₄-haloalkynyl; and
in case b), if at least one of the groups R², R³, R⁴ and R⁵ is not hydrogen:
X is phenyl, naphthyl or a five- or six-membered saturated, partially unsatu- rated or aromatic heterocycle which is attached via a carbon atom and con- tains one to four heteroatoms selected from the group consisting of O, N and S, where X may carry one to four groups R^{a}:
R^{a} is halogen, cyano, nitro, C₁-C₈-alkyl, C₁-C₈-alkoxy, C₁-C₈-haloalkoxy, C₁-C₄-alkylcarbonyl, C₁-C₄-alkoxycarbonyl, -C(R⁶)=NOR⁷, C₁-C₄-alkylaminocarbonyl, di(C₁-C₄-alkyl)aminocarbonyl or phenyl or phenoxy, where the rings may carry one to three groups R^{b}:
R^{b} is halogen, C₁-C₄-alkyl, C₁-C₄-alkoxy, C₁-haloalkyl or C₁-haloalkoxy;
R^{a} or R^{b} may also be a C₃-C₄-alkylene or a C₄-alkenylene group which, together with two adjacent ring members of the phenyl ring to which it is attached, forms a ring which may be substi- tuted by one or more of the abovementioned groups R^{a} or R^{b}.

2. The compound of the formula I according to claim 1, in which R², R³, R⁴ and R⁵ independently of one another are hydrogen, methyl, fluorine, chlorine, CF₃, OCF₃ or OCHF₂.

3. The compound of the formula I according to claim 1 or 2 in which R¹ is hydrogen, methyl, methoxy, ethoxy, allyl or propargyl.

4. The compound of the formula I according to any of claims 1 to 3 in which X is a phenyl ring which is substituted in the para position.

5. The compound of the formula I according to any of claims 1 to 3 in which X is an aromatic heterocycle.

6. A method for preparing a compound of the formula I according to any of claims 1 to 5 by reacting pyridine derivatives of the formula II in which the variables are as defined in claim 1 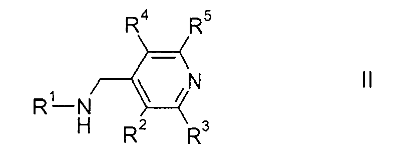 under basic conditions with a sulfonyl chloride of the formula III, in which X is as defined in claim 1,

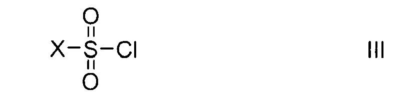

7. A composition suitable for controlling harmful fungi, which composition comprises a solid or liquid carrier and a compound of the formula I according to claim 1.

8. A method for controlling phytopathogenic harmful fungi, which method comprises treating the fungi or the materials, plants, the soil or seeds to be protected against fungal attack with an effective amount of a compound of the formula I according to claim 1.

9. A pyridine derivative of the formula II according to claim 6, where the substituents are as follows:
R¹ is hydrogen or methyl;
R⁴, R⁵ are hydrogen; and
R² = R³ are both methyl, fluorine, chlorine, methoxy or trifluoromethoxy.

10. A pyridine derivative of the formula II according to claim 6, where the substituents are as follows:
R¹ is hydrogen or methyl;
R², R⁴ are hydrogen; and
R³ = R⁵ are both fluorine, methoxy or trifluoromethoxy.

11. A pyridine derivative of the formula II according to claim 6, where the substituents are as follows:
R¹ is hydrogen or methyl;
R³, R⁵ are hydrogen; and
R² = R⁴ are both methyl, fluorine, chlorine, methoxy or trifluoromethoxy.

12. A pyridine derivative of the formula II according to any of claims 9 to 11 in which R¹ is hydrogen.

## Revendications

1. Sulfonamides de formule I 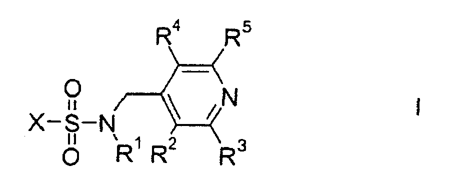 dans laquelle les substituants ont les significations suivantes :
R¹ représente un atome d'hydrogène, un groupe alkyle en C₁-C₄, alcoxy en C₁-C₄, alcényle en C₂-C₄, alcynyle en C₂-C₄ ou benzyle ;
R², R³, R⁴, R⁵ représentent, indépendamment les uns des autres, un atome d'hydrogène ou d'halogène ou un groupe alkyle en C₁-C₄, alcoxy en C₁-C₄ ou halogénométhyle en C₁ ;
R² et R³ peuvent également former ensemble un cycle phényle, cyclopentyle ou cyclohexyle, ces cycles pouvant porter deux groupes R^{2'} et R^{3'} ,
R^{2'}, R^{3'} représentent, indépendamment l'un de l'autre, un atome d'hydrogène ou d'halogène ou un groupe alkyle en C₁-C₄, alcoxy en C₁-C₄ ou halogénométhyle en C₁ ;
dans le cas a) où R², R³, R⁴ et R⁵ représentent des atomes d'hydrogène:
X représente un cycle phényle qui est substitué par un groupe -C (R⁶)=NOR⁷, où
R⁶ représente un groupe alkyle en C₁-C₄ et
R⁷ un groupe alkyle en C₁-C₈, benzyle, alcényle en C₂-C₄, halogénoalkyle en C₁-C₄, halogénoalcényle en C₂-C₄, alcynyle en C₂-C₄ ou halogénoalcynyle en C₂-C₄ ; et
dans le cas b) où au moins l'un des groupes R², R³, R⁴ et R⁵ ne représente pas un atome d'hydrogène :
X représente un cycle phényle, naphtyle ou un hétérocycle saturé, partiellement insaturé ou aromatique à cinq ou six chaînons, lié par un atome de carbone, comportant un à quatre hétéroatomes choisis parmi O, N et S, X pouvant porter jusqu'à quatre groupes R^{a} :
R^{a} représentant un atome d'halogène, un groupe cyano, nitro, alkyle en C₁-C₈, alcoxy en C₁-C₈, halogénoalcoxy en C₁-C₈, alkyl(C₁-C₄)carbonyle, alcoxy(C₁-C₄)carbonyle, C(R⁶) =NOR⁷, alkyl(C₁-C₄)amino-carbonyle, di [alkyl (C₁-C₄)] amino-carbonyle ou phényle ou phénoxy, les cycles pouvant porter un à trois groupes R^{b} :
R^{b} représentant un atome d'halogène, un groupe alkyle en C₁-C₄, alcoxy en C₁-C₄, halogénoalkyle en C₁ ou halogénoalcoxy en C₁ ;
R^{a} ou R^{b} pouvant également représenter un groupe alkylène en C₃-C₄ ou un groupe alcénylène en C₄, formant avec deux chaînons contigus du cycle phényle auxquels il est lié, un cycle qui peut être substitué par un ou plusieurs des groupes R^{a} ou R^{b} nommés précédemment.

2. Composés de formule I selon la revendication 1, dans lesquels R², R³, R⁴ et R⁵ représentent, indépendamment les uns des autres un atome d'hydrogène, de fluor ou de chlore, ou un groupe méthyle, CF₃ OCF₃ ou OCHF₂.

3. Composés de formule I selon la revendication 1 ou 2, dans lesquels R¹ représente un atome d'hydrogène, le groupe méthyle, méthoxy, éthoxy, allyle ou propargyle.

4. Composés de formule I selon l'une quelconque des revendications 1 à 3, dans lesquels X représente un cycle phényle qui est substitué en position para.

5. Composés de formule I selon l'une quelconque des revendications 1 à 3, dans lesquels X représente un hétérocycle aromatique.

6. Procédé pour la préparation des composés de formule I selon l'une quelconque des revendications 1 à 5, par mise en réaction, dans des conditions basiques, de dérivés de pyridine de formule II, 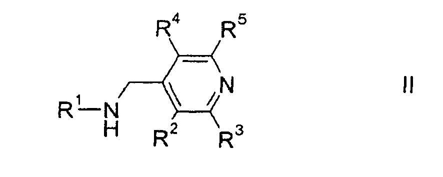 dans laquelle les variables sont définies selon la revendication 1,
avec des chlorures de sulfonyle de formule III, 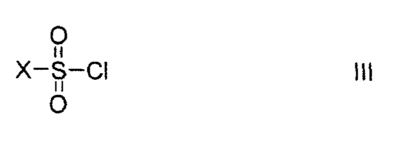 dans laquelle X a la signification selon la revendication 1.

7. Composition appropriée à la lutte contre des champignons nuisibles, contenant une matière de support solide ou liquide et un composé de formule I selon la revendication 1.

8. Procédé pour la lutte contre des champignons nuisibles phytopathogènes, **caractérisé en ce qu'**on traite par une quantité efficace d'un composé de formule I selon la revendication 1 les champignons ou les matériaux, les plantes, le sol ou les semences, à protéger contre l'attaque de champignons.

9. Dérivés de pyridine de formule II selon la revendication 6, dans lesquels les substituants ont les significations suivantes :
R¹ représente un atome d'hydrogène ou le groupe méthyle ;
R⁴, R⁵ représentent un atome d'hydrogène ; et
R² = R³ représentent l'un et l'autre un atome de fluor ou de chlore ou le groupe méthyle, méthoxy ou trifluorométhoxy.

10. Dérivés de pyridine de formule II selon la revendication 6, dans lesquels les substituants ont les significations suivantes :
R¹ représente un atome d'hydrogène ou le groupe méthyle ;
R², R⁴ représentent un atome d'hydrogène ; et
R³ = R⁵ représentent l'un et l'autre un atome de fluor ou le groupe méthoxy ou trifluorométhoxy.

11. Dérivés de pyridine de formule II selon la revendication 6, dans lesquels les substituants ont les significations suivantes :
R¹ représente un atome d'hydrogène ou le groupe méthyle ;
R³, R⁵ représentent un atome d'hydrogène ; et
R² = R⁴ représentent l'un et l'autre un atome de fluor ou le groupe méthyle, méthoxy ou trifluorométhoxy.

12. Dérivés de pyridine de formule II selon l'une quelconque des revendications 9 à 11, dans lesquelles R¹ représente un atome d'hydrogène.
